(19) Europäisches Patentamt
European Patent Office
Office européen des brevets

(11) Veröffentlichungsnummer: **0 030 343**
**B1**

(12) # EUROPÄISCHE PATENTSCHRIFT

(45) Veröffentlichungstag der Patentschrift: **31.08.83**

(51) Int. Cl.³: **C 07 D 211/84,** C 07 D 405/04, C 07 D 409/04, A 61 K 31/445

(21) Anmeldenummer: **80107492.3**

(22) Anmeldetag: **30.11.80**

(54) **Substituierte 2-Amino-3,4-dihydropyridinderivate, Verfahren zu ihrer Herstellung und ihre Verwendung als Arzneimittel.**

(30) Priorität: **11.12.79 DE 2949701**
**02.05.80 DE 3016874**

(43) Veröffentlichungstag der Anmeldung: **17.06.81 Patentblatt 81/24**

(45) Bekanntmachung des Hinweises auf die Patenterteilung: **31.08.83 Patentblatt 83/35**

(84) Benannte Vertragsstaaten: **AT BE CH DE FR GB IT LI NL SE**

(56) Entgegenhaltungen: **LU-A-68 206**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Meyer, Horst, Dr., Theodor-Heuss-Strasse 110, D-5600 Wuppertal 1 (DE)**
Erfinder: **Sitt, Rüdiger, Dr., Am Jagdhaus 116b, D-5600 Wuppertal 1 (DE)**
Erfinder: **Thomas, Günter, Dr., Claudiusweg 9, D-5600 Wuppertal 1 (DE)**
Erfinder: **Garthoff, Bernward, Dr., Haendelstrasse 22, D-4010 Hilden (DE)**
Erfinder: **Towart, Robertson, Dr., Claudiusweg 9, D-5600 Wuppertal 1 (DE)**
Erfinder: **Rosentreter, Ulrich, Dr., Waldstrasse 11, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Substituierte 2-Amino-3,4-dihydropyridinderivate, Verfahren zu ihrer Herstellung und ihre Verwendung als Arzneimittel

Die Erfindung betrifft neue substituierte 2-Amino-3,4-dihydropyridinderivate, ein Verfahren zu ihrer Herstellung sowie ihre Verwendung als Arzneimittel, insbesondere ihre Verwendung als Lipidabsorptionshemmer.

Es ist bereits bekannt, dass 3,5-Bisalkoxycarbonyl-2-alkylaminodihydropyridine kreislaufbeeinflussende Wirkungen, insbesondere blutdrucksenkende Wirkung besitzen (vgl. DT-OS Nr. 2239815). Eine lipidabsorptionshemmende Wirkung von Dihydropyridinen ist bisher noch nicht bekannt geworden.

Die Erfindung betrifft neue substituierte 2-Amino-3,4-dihydropyridinderivate der allgemeinen Formel I

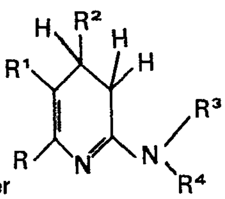

(I)

in welcher

R für geradkettiges oder verzweigtes Alkyl mit bis zu 6 Kohlenstoffatomen steht, welches gegebenenfalls 1- oder 2fach substituiert ist durch Hydroxy, Alkoxy mit 1 bis 4 Kohlenstoffatomen, Amino oder Dialkylamino mit jeweils 1 bis 4 Kohlenstoffatomen, oder für einen Phenyl- oder Benzylrest steht, wobei der Phenylring gegebenenfalls 1- oder 2fach substituiert ist durch gleiche oder verschiedene Substituenten aus der Gruppe Fluor, Chlor, Brom, Trifluormethyl, Alkyl oder Alkoxy mit jeweils 1 bis 4 Kohlenstoffatomen,

$R^1$ und $R^2$ gleich oder verschieden sind und jeweils für Phenyl, Naphthyl, Pyridyl, Furyl oder Thienyl stehen, wobei diese aromatischen Ringe gegebenenfalls durch 1 oder 2 gleiche oder verschiedene Substituenten aus der Gruppe Nitro, Cyano, Hydroxy, Amino, Carboxy, Halogen, Phenyl, Alkyl, Alkoxy, Trifluormethyl, Trifluormethoxy, Trifluormethylthio, Alkylmercapto, Alkylsulfonyl, Alkylamino oder Dialkylamino substituiert sind, wobei die genannten Alkyl- und Alkoxyreste jeweils 1 bis 4 Kohlenstoffatome enthalten, und

$R^3$ und $R^4$ gleich oder verschieden sind und jeweils für Wasserstoff, Benzyl oder Alkyl mit bis zu 6 Kohlenstoffatomen stehen, oder gemeinsam mit dem Stickstoffatom einen 5- bis 7gliedrigen Ring bilden, der gegebenenfalls durch Sauerstoff, NH oder $NR^6$ unterbrochen ist, wobei $R^6$ die für $R^1$ und $R^2$ angegebene Bedeutung besitzt,

sowie ihre pharmazeutisch unbedenklichen Additionssalze.

Weiterhin wurde gefunden, dass man Verbindungen der allgemeinen Formel I erhält, wenn man α,β-ungesättigte Oxoverbindungen der allgemeinen Formel II

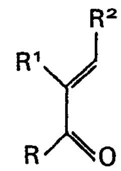

(II)

in welcher

R, $R^1$ und $R^2$ die oben angegebene Bedeutung haben, mit einem 3,3-Diaminoacrylsäureester der allgemeinen Formel IIIa

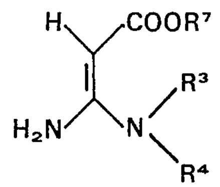

(IIIa)

bzw. ihrer tautomeren Iminform der allgemeinen Formel IIIb

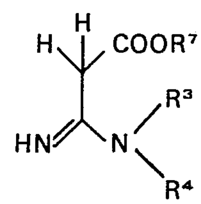

(IIIb)

in welcher

$R^3$ und $R^4$ die oben angegebene Bedeutung haben, und

$R^7$ für einen gegebenenfalls substituierten Alkyl- oder Aralkylrest steht,

in Gegenwart von inerten organischen Lösungsmitteln und gegebenenfalls in Gegenwart von sauren oder basischen Katalysatoren bei Temperaturen zwischen 20 und 150°C umsetzt.

Verwendet man 3-(p-Chlorphenyl)-4-α-pyridylbuten-3-on-2 und 3-Amino-3-$N^4$-(p-trifluormethylphenyl)piperazinoacrylsäureäthylester als Ausgangsverbindungen, so kann der Reaktionsablauf durch folgendes Formelschema wiedergegeben werden:

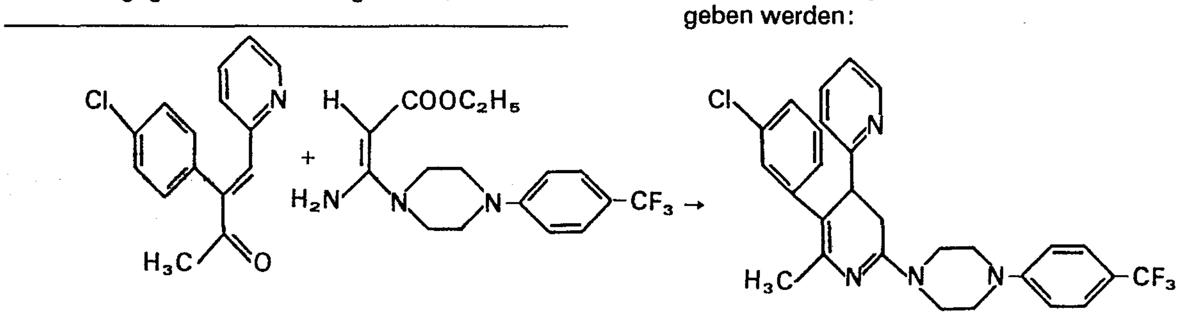

Die erfindungsgemäss einsetzbaren α,β-ungesättigten Oxoverbindungen der allgemeinen Formel II sind bekannt oder können nach bekannten Methoden hergestellt werden [vgl. „J. Am. Chem. Soc." *81*, 113 (1959)].

Die als Ausgangsverbindungen verwendbaren 3,3-Diaminoacrylsäureester der allgemeinen Formel IIIa bzw. ihre tautomeren Iminformen sind ebenfalls bekannt oder können nach bekannten Methoden hergestellt werden (vgl. DT-OS Nr. 2239815 und H. Meyer, F. Bossert, H. Horstmann, „Liebigs Ann. Chem." *1977*, 1895).

Bei der Durchführung des erfindungsgemässen Verfahrens kommen als Verdünnungsmittel alle inerten organischen Lösungsmittel in Frage. Hierzu gehören vorzugsweise niedere Alkohole wie Methanol, Äthanol, Propanol; Äther wie Dioxan, Diäthyläther; Ketone wie Aceton; Eisessig, Pyridin, Dimethylformamid, Dimethylsulfoxid oder Acetonitril.

Die Reaktionstemperaturen können in einem grösseren Bereich variiert werden. Im allgemeinen arbeitet man zwischen 20 und 150°C, vorzugsweise bei 30 bis 120°C, insbesondere bei der Siedetemperatur des Lösungsmittels.

Als saure Katalysatoren seien vorzugsweise genannt: Mineralsäuren, wie Schwefelsäure oder Phosphorsäure, Sulfonsäure wie Toluolsulfonsäure und als basische Katalysatoren seien vorzugsweise genannt Alkali- und Erdalkalihydroxide und Alkoholate, insbesondere Alkalimetallalkoholate.

Die Umsetzung kann bei Normaldruck, aber auch bei erhöhtem Druck, durchgeführt werden. Im allgemeinen arbeitet man bei Normaldruck.

Bei der Durchführung des Verfahrens werden die an der Reaktion beteiligten Stoffe vorzugsweise jeweils in molaren Mengen eingesetzt.

Von besonderem Interesse sind Verbindungen der allgemeinen Formel I, in welcher

R für Alkyl mit 1 bis 4 Kohlenstoffatomen, Benzyl oder Phenyl steht,

$R^1$ und $R^2$ gleich oder verschieden sind und jeweils für Phenyl, Naphthyl, Pyridyl, Furyl oder Thienyl stehen, wobei diese aromatischen Ringe gegebenenfalls durch 1 oder 2 gleiche oder verschiedene Substituenten aus der Gruppe Cyano, Hydroxy, Carboxy, Halogen, Phenyl, Alkyl, Alkoxy, Trifluormethyl, Trifluormethoxy, Trifluormethylthio, Alkylmercapto, Alkylsulfonyl, Alkylamino oder Dialkylamino substituiert sind, wobei die genannten Alkyl- und Alkoxyreste jeweils 1 bis 4 Kohlenstoffatome enthalten, und

$R^3$ und $R^4$ gleich oder verschieden sind und jeweils für Wasserstoff oder Alkyl mit bis zu 6 Kohlenstoffatomen stehen, oder gemeinsam einen 5- bis 7gliedrigen Ring bilden, der gegebenenfalls durch Sauerstoff, NH oder $NR^6$ unterbrochen ist, wobei $R^6$ die für $R^1$ und $R^2$ angegebene Bedeutung besitzt,

sowie ihre pharmazeutisch unbedenklichen Additionssalze.

Überraschenderweise zeigen die erfindungsgemässen Verbindungen der allgemeinen Formel I eine sehr starke Wirkung bei der Behandlung von Fettstoffwechselstörungen. Sie bewirken insbesondere eine Senkung des erhöhten Cholesterins im Serum und vermindern gleichzeitig eine Hypertriglycerinämie.

Die erfindungsgemässen Verbindungen eignen sich daher vorteilhaft zur Behandlung von Hyperlipoproteinämien, Atherosklerose, Adipositas und zur Behandlung hierdurch ausgelöster Stoffwechselstörungen.

Die erfindungsgemässen Verbindungen sind besonders geeignet als Lipidabsorptionshemmer, Diuretika, Saluretika, Antiarrythmika und Cardiotonika.

Die diuretische und saluretische Wirkung wird an Ratten untersucht. Hierzu werden männliche Ratten verwendet, die in nüchternem Zustand 10 ml/kg Flüssigkeit per Schlundsonde erhalten. Diese Flüssigkeit enthält 0,5 Tylose sowie eine bestimmte Dosis des Prüfpräparates (Kontrolltiere ohne Prüfpräparat). Der ausgeschiedene Harn wird 6 h gesammelt und anschliessend der Natrium- und Kaliumgehalt in üblicher Weise photometrisch bestimmt.

Die antiarrythmische Wirkung der erfindungsgemässen Verbindungen wird durch die Einflüsse auf die Refraktärzeit der Herzmuskulatur mittels Standardtestverfahren nachgewiesen. Bekanntlich verlängern Antiarrythmika in therapeutischen Dosen die Refraktärzeit der Herzmuskulatur. Diese Verlängerung der Refraktärzeit sowie die Bestimmung der Kontraktionskraft an isolierten Herzmuskel-Präparaten erfolgt nach bekannten Methoden [vgl.: Govier „J. Pharmacol. Exp. Ther." *148*, 100-105 (1965) und Roseblueth *et al.*, „J. Cell. Comp. Physiol." *33*, 405-439 (1949)].

Aus der Stoffklasse der Dihydropyridine sind bisher keine dieser Wirkungen bekannt geworden. Es ist daher als ausgesprochen überraschend zu bezeichnen, dass die erfindungsgemässen Verbindungen diese neuen und vorteilhaften Wirkungen besitzen. Als neuartige Stoffklasse zur Behandlung von Stoffwechselstörungen und Herzrythmusstörungen, bei gleichzeitig sehr guter Verträglichkeit, stellen sie eine Bereicherung der Pharmazie dar.

Zur Erfindung gehören auch pharmazeutische Zubereitungen, die neben nichttoxischen, inerten pharmazeutisch geeigneten Trägerstoffen eine oder mehrere Verbindungen der allgemeinen Formel I enthalten oder die aus einer oder mehreren Verbindungen der allgemeinen Formel I bestehen, sowie Verfahren zur Herstellung dieser Zubereitungen.

Zur Erfindung gehören auch pharmazeutische Zubereitungen in Dosierungseinheiten. Dies bedeutet, dass die Zubereitungen in Form einzelner Teile, z.B. Tabletten, Dragées, Kapseln, Pillen, Suppositorien und Ampullen, vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder ½, ⅓ oder ¼ einer Einzeldosis enthalten. Eine Einzeldosis enthält vorzugsweise die Menge Wirkstoff, die bei einer Applikation verabreicht wird und die gewöhnlich einer ganzen, einer

halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragées, Kapseln, Pillen, Granulate, Suppositorien, Lösungen, Suspensionen und Emulsionen, genannt.

Tabletten, Dragées, Kapseln, Pillen und Granulate können den oder die Wirkstoffe neben den üblichen Trägerstoffen enthalten, wie a) Füll- und Streckmittel, z.B. Stärken, Milchzucker, Rohrzucker, Glukose, Mannit und Kieselsäure, b) Bindemittel, z.B. Carboxymethylcellulose, Alginate, Gelatine, Polyvinylpyrrolidon, c) Feuchthaltemittel, z.B. Glycerin, d) Sprengmittel, z.B. Agar-Agar, Calciumcarbonat und Natriumbicarbonat, e) Lösungsverzögerer, z.B. Paraffin, f) Resorptionsbeschleuniger, z.B. quarternäre Ammoniumverbindungen, g) Netzmittel, z.B. Cetylalkohol, Glycerinmonostearat, h) Adsorptionsmittel, z.B. Kaolin und Bentonit, und i) Gleitmittel, z.B. Talkum-, Calcium- und Magnesiumstearat und feste Polyäthylenglykole oder Gemische der unter a) bis i) aufgeführten Stoffe.

Die Tabletten, Dragées, Kapseln, Pillen und Granulate können mit den üblichen, gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes, gegebenenfalls verzögert, abgeben, wobei als Einbettungsmassen, z.B. Polymersubstanzen und Wachse, verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Lösungen und Emulsionen können neben dem oder den Wirkstoffen die üblichen Trägerstoffe, wie Lösungsmittel, Lösungsvermittler und Emulgatoren, z.B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Rizinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Äthylalkohol, Propylenglykol, Suspendiermittel, z.B. äthoxylierte Isostearylalkohole, Polyoxyäthylensorbit- und -sorbitanester, mikrokristalline Cellulose, Aluminiummethanhydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süssmittel, z.B. Saccharin, enthalten.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.% der Gesamtmischung vorhanden sein.

Die oben aufgeführten pharmazeutischen Zubereitungen können ausser Verbindungen der allgemeinen Formel I auch noch andere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können oral, parenteral, intraperitoneal und/oder rektal, vorzugsweise oral, appliziert werden.

Im allgemeinen hat es sich sowohl in der Human- als auch in der Veterinärmedizin als vorteilhaft erwiesen, den oder die Wirkstoffe in Mengen von etwa 1,0 bis etwa 500, vorzugsweise 5 bis 100 mg/kg Körpergewicht je 24 h, verteilt auf 1 bis 6 Verabreichungen und zwar vor oder/und während oder/und nach der Mahlzeit zu applizieren. Eine Einzelgabe enthält den oder die Wirkstoffe, vorzugsweise in Mengen von etwa 1 bis etwa 100 mg/kg Körpergewicht. Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verarbreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

*Ausführungsbeispiele*

*Beispiel 1:*

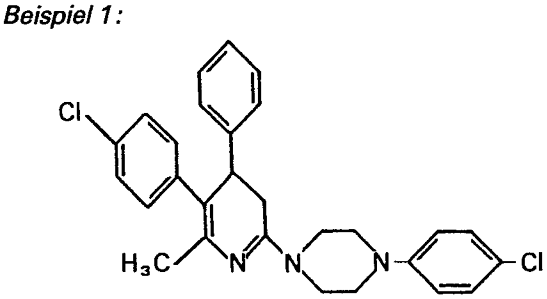

0,033 mol 3-(p-Chlorphenyl)-4-phenylbuten-3-on-2 und 0,033 mol 3-Amino-3-$N^4$-(p-chlorphenyl)piperazinoacrylsäureäthylester werden in 100 ml Äthanol 30 h unter Rückfluss gekocht. Nach Abkühlen der Reaktionsmischung wird der ausgefallene Niederschlag abgesaugt und mit Äthanol gewaschen. Nach Umkristallisation aus Äthanol erhält man 5-(4-Chlorphenyl)-2-(4-chlorphenylpiperazino)-6-methyl-4-phenyl-3,4-dihydropyridin vom Schmelzpunkt 161-162°C.

Ausbeute: 48% der Theorie.

Die Beispiele der nachfolgenden Tabelle wurden analog Beispiel 1 hergestellt:

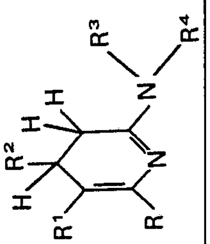

Tabelle 1

| Beispiel-Nr. | R | $R_1$ | $R_2$ | $R_3$ und $R_4$ | Umkristallisiert aus Lösungsmittel | Fp. (°C) | Ausbeute (% d. Th.) |
|---|---|---|---|---|---|---|---|
| 2 | $CH_3$ | 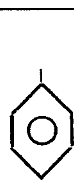 | 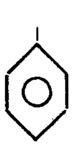 | $(-C_2H_4)_2N-$ 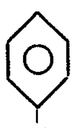 | Äthanol | 103-104 | 17 |
| 3 | $CH_3$ | 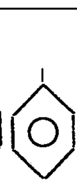 | 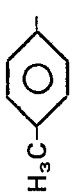 | $(-C_2H_4)_2N-$ 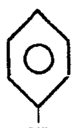 | Äthanol | 142-144 | 16 |
| 4 | $CH_3$ | 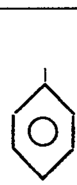 | 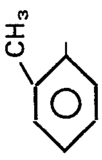 | $(-C_2H_4)_2N-$ 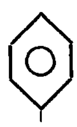 | Äther | 124-126 | 25 |
| 5 | $CH_3$ | 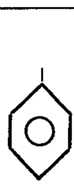 | 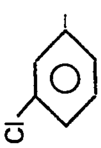 | $(-C_2H_4)_2N-$ 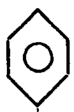 | Äthanol | 101-105 | 48 |
| 6 | $CH_3$ | 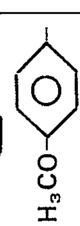 | 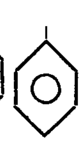 | $(-C_2H_4)_2N-$ 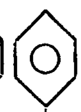 | Äthanol | 132-134 | 16 |
| 7 | $CH_3$ | 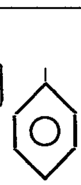 | 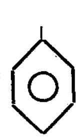 | $(-C_2H_4)_2N-$ 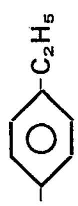 | Äthanol | 97-98 | 21 |
| 8 | $CH_3$ | 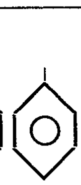 | 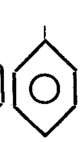 | $(-C_2H_4)_2N-$ 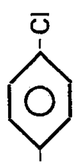 | Äthanol | 106-108 | 13 |
| 9 | $CH_3$ | 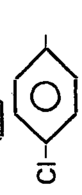 | 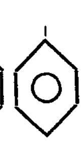 | $(-C_2H_4)_2N-$ 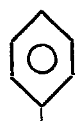 | Dioxan | 180-181 | 69 |
| 10 | $CH_3$ | 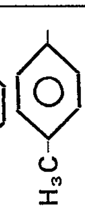 | 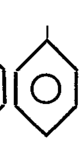 | $(-C_2H_4)_2N-$ 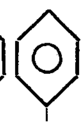 | Äthanol | 130-132 | 40 |
| 11 | $CH_3$ | 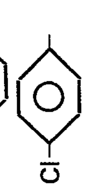 | 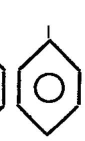 | $(-C_2H_4)_2N-$ 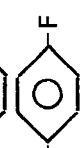 | Äthanol | 155-157 | 43 |
| 12 | $CH_3$ | 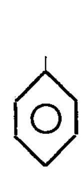 | 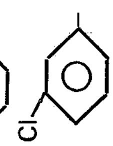 | $(-C_2H_4)_2N-$ 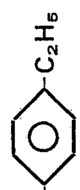 | Äthanol | 122-124 | 43 |

Tabelle 1 *(Fortsetzung)*

| Beispiel-Nr. | R | $R_1$ | $R_2$ | $R_3$ und $R_4$ | Umkristallisiert aus Lösungsmittel | Fp. (°C) | Ausbeute (% d. Th.) |
|---|---|---|---|---|---|---|---|
| 13 | $CH_3$ | 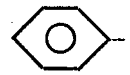 | 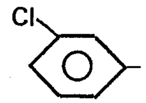 | 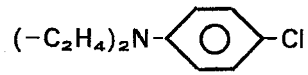 | Äthanol | 124-131 | 45 |
| 14 | $CH_3$ | 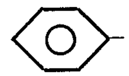 | 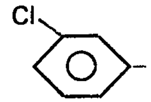 | 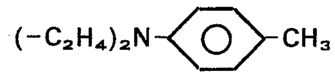 | Äthanol | 120-121 | 57 |
| 15 | $CH_3$ | 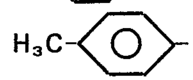 | 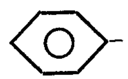 | 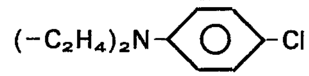 | Äthanol | 137-138 | 37 |
| 16 | $CH_3$ | 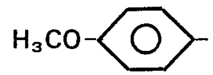 | 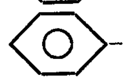 | 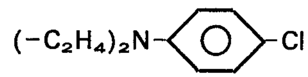 | Äthanol | 137-138 | 39 |
| 17 | $CH_3$ | 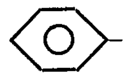 | 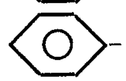 | 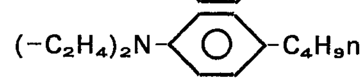 | Methanol × 2HCl | 223-224 | 23 |
| 18 | $CH_3$ | 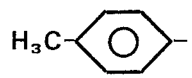 | 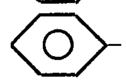 | 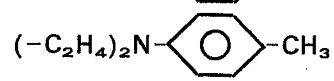 | Äthanol | 100-102 | 35 |
| 19 | $CH_3$ | 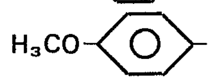 | 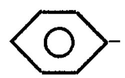 | 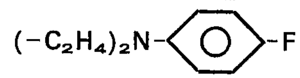 | Äthanol | 118-119 | 43 |
| 20 | $CH_3$ | 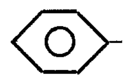 | 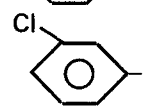 | 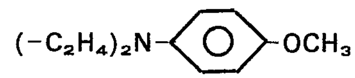 | Äthanol | 112 | 38 |
| 21 | $CH_3$ | 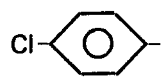 | 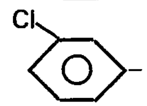 | 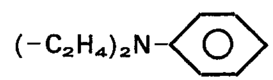 | Äthanol | 114 | 65 |
| 22 | $CH_3$ | 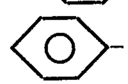 | 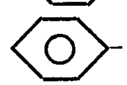 | 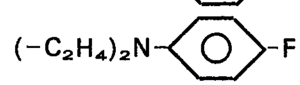 | Methanol × 2HCl | 242-243 | 21 |
| 23 | $CH_3$ | 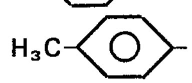 | 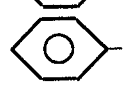 | 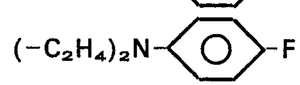 | Äthanol | 104-105 | 31 |
| 24 | $CH_3$ | 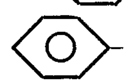 | 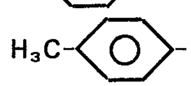 | 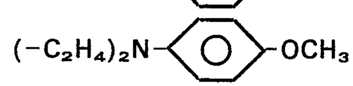 | Äthanol | 138 | 48 |
| 25 | $CH_3$ | 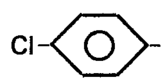 | 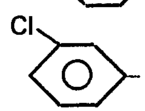 | 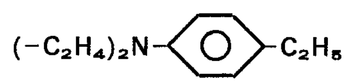 | Äthanol | 98 | 54 |

Tabelle 1 *(Fortsetzung)*

| Beispiel-Nr. | R | $R_1$ | $R_2$ | $R_3$ und $R_4$ | Umkristallisiert aus Lösungsmittel | Fp. (°C) | Ausbeute (% d. Th.) |
|---|---|---|---|---|---|---|---|
| 26 | $CH_3$ | 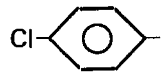 | 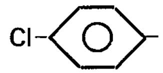 | $(-C_2H_4)_2N-$ 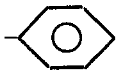 | Äthanol | 140 | 38 |
| 27 | $CH_3$ | 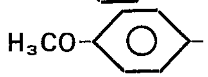 | 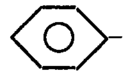 | $(-C_2H_4)_2N-$ 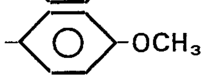 | Äthanol | 122 | 49 |
| 28 | $CH_3$ | 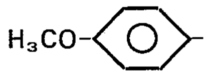 | 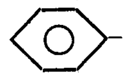 | $(-C_2H_4)_2N-$ 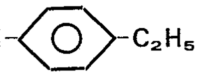 | Äthanol | 94 | 33 |
| 29 | $CH_3$ | 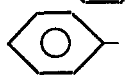 | 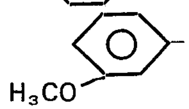 | $(-C_2H_4)_2N-$ 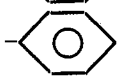 | Methanol × 2HCl | 139 | 25 |
| 30 | $CH_3$ | 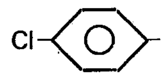 | 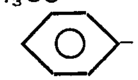 | $(-C_2H_4)_2N-$ 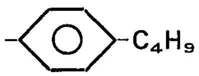 | Äthanol | 48 | 24 |
| 31 | $CH_3$ | 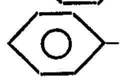 | 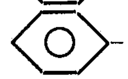 | H H | Cyclohexan | 164 | 19 |
| 32 | $CH_3$ | 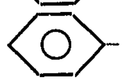 | 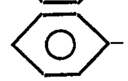 | $-(CH_2)_4-$ | Äther | 116 | 52 |
| 33 | $CH_3$ | 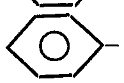 | 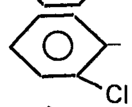 | $-(CH_2)_4-$ | Äthanol | 144 | 57 |
| 34 | $CH_3$ | 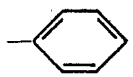 | 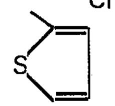 | $(-C_2H_4)_2N-$ 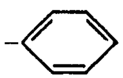 | Äther | 143 | 33 |
| 35 | $CH_3$ | 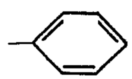 | 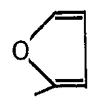 | $(-C_2H_4)_2N-$ 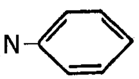 | Äthanol | 125 | 40 |
| 36 | $CH_3$ | 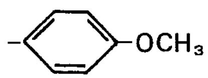 | 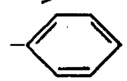 | $-(CH_2)_4-$ | Isopropanol | 139 | 53 |
| 37 | $CH_3$ | 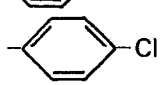 | 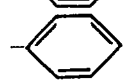 | $-(CH_2)_4-$ | Isopropanol | 152 | 52 |
| 38 | $CH_3$ | 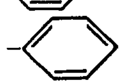 | 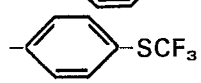 | $(-C_2H_4)_2N-$ 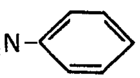 | Methanol/Aceton ×2HCl | 225-227 | 33 |
| 39 | $CH_3$ | 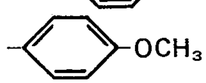 | 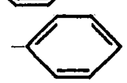 | $(-C_2H_4)_2N-$ 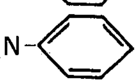 | Äthanol | 132-134 | 15 |

Tabelle 1 *(Fortsetzung)*

| Beispiel-Nr. | R | $R_1$ | $R_2$ | $R_3$ und $R_4$ | Umkristallisiert aus Lösungsmittel | Fp. (°C) | Ausbeute (% d. Th.) |
|---|---|---|---|---|---|---|---|
| 40 | $CH_3$ | 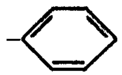 | 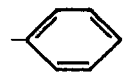 | $(-C_2H_4)_2N-$ 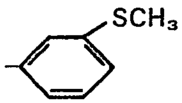 | Methanol/Aceton ×2HCl | 202-204 | 41 |
| 41 | $CH_3$ | 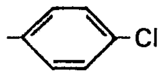 | 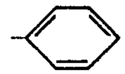 | $(-C_2H_4)_2N-$ 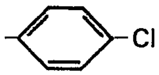 | Äthanol | 161-163 | 45 |
| 42 | $CH_3$ | 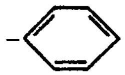 | 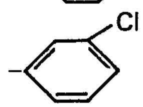 | $(-C_2H_4)_2N-$ 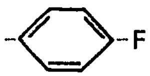 | Methanol/Aceton ×2HCl | 202-205 | 35 |
| 43 | $CH_3$ | 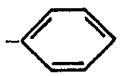 | 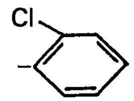 | $(-C_2H_4)_2N-$ 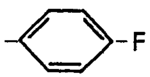 | Methanol/Aceton ×2HCl | 202-210 (Zers.) | 20 |
| 44 | $CH_3$ | 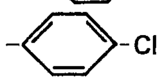 | 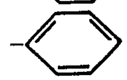 | $(-C_2H_4)_2N-$ 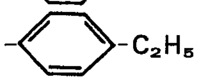 | Methanol/Aceton ×2HCl | 243-244 | 39 |
| 45 | $CH_3$ | 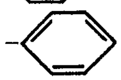 | 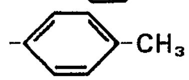 | $(-C_2H_4)_2N-$ 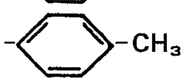 | Methanol/Aceton ×2HCl | 225-227 | 38 |
| 46 | $CH_3$ | 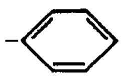 | 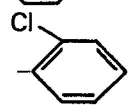 | $(-C_2H_4)_2N-$ 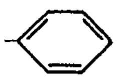 | Methanol/Aceton ×2HCl | 221-223 | 18 |
| 47 | $CH_3$ | 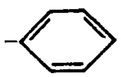 | 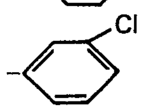 | $(-C_2H_4)_2N-$ 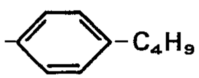 | Methanol/Äther ×2HCl | 225-227 | 37 |
| 48 | $CH_3$ | 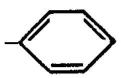 | 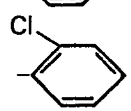 | $(-C_2H_4)_2N-$ 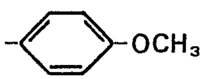 | Methanol/Äther ×2HCl | 221-223 | 17 |
| 49 | $CH_3$ | 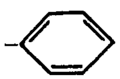 | 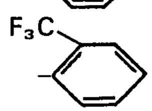 | $(-C_2H_4)_2N-$ 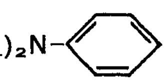 | Methanol/Äther ×2HCl | 173-176 | 14 |
| 50 | $CH_3$ | 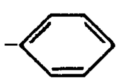 | 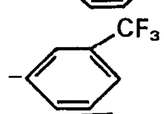 | $(-C_2H_4)_2N-$ 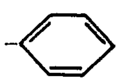 | Methanol/Äther ×2HCl | 234-236 | 47 |
| 51 | $CH_3$ | 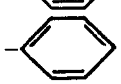 | 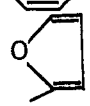 | $(-C_2H_4)_2N-$ 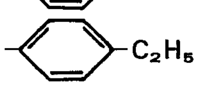 | Methanol/Äther ×2HCl | 233-235 | 36 |

Tabelle 1 *(Fortsetzung)*

| Beispiel-Nr. | R | $R_1$ | $R_2$ | $R_3$ und $R_4$ | Umkristallisiert aus Lösungsmittel | Fp. (°C) | Ausbeute (% d. Th.) |
|---|---|---|---|---|---|---|---|
| 52 | $CH_3$ | 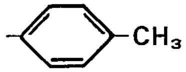 | 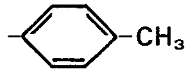 | $(-C_2H_4)_2N-$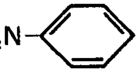 | Cyclohexan | 126-131 | 15 |
| 53 | $CH_3$ | 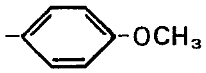 | 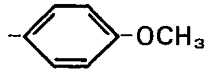 | $(-C_2H_4)_2N-$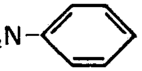 | Äthanol | 148 | 17 |
| 54 | $CH_3$ | 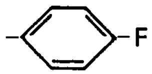 | 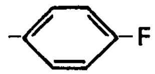 | $(-C_2H_4)_2N-$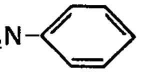 | Äthanol | 146-148 | 47 |
| 55 | $CH_3$ | 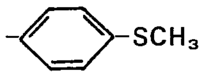 | 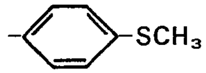 | $(-C_2H_4)_2N-$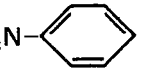 | Äthanol | 149-151 | 37 |
| 56 | $CH_3$ | 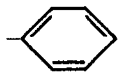 | 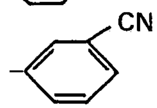 | $(-C_2H_4)_2N-$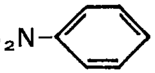 | Petroläther | 58-63 | 70 |
| 57 | $CH_3$ | 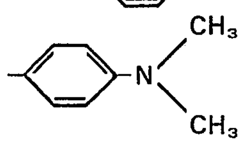 | 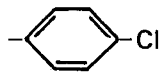 | $(-C_2H_4)_2N-$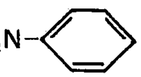 | Äthanol | 190 | 40 |
| 58 | $CH_3$ | 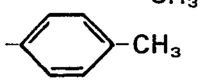 | 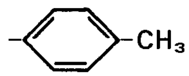 | $-(CH_2)_4-$ | Cyclohexan | 99 | 58 |
| 59 | $CH_3$ | 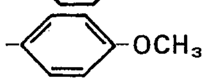 | 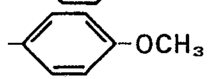 | $-(CH_2)_4-$ | Äther | 143 | 54 |
| 60 | $CH_3$ | 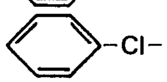 | 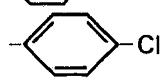 | $-(CH_2)_4-$ | Cyclohexan | 137 | 47 |
| 61 | $CH_3$ | 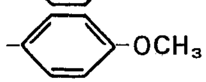 | 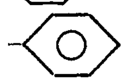 | $-(CH_2)_4-$ | Isopropanol | 139-140 | 53 |
| 62 | $CH_3$ | 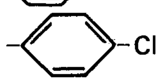 | 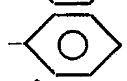 | $-(CH_2)_4-$ | Isopropanol | 152-154 | 52 |
| 63 | $CH_3$ | 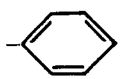 | 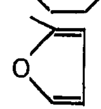 | $(-C_2H_4)_2N-$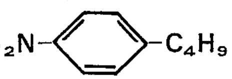 | Methanol/Aceton $\times 2HCl$ | 224-226 | 44 |
| 64 | $CH_3$ | 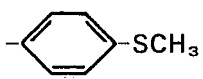 | 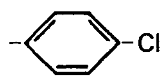 | $(-C_2H_4)_2N-$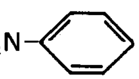 | Äthanol | 164 | 37 |
| 65 | $CH_3$ | 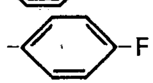 | 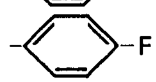 | $-(CH_2)_4-$ | Äthanol × HCl | 70 | 46 |

Tabelle 1 *(Fortsetzung)*

| Beispiel-Nr. | R | $R_1$ | $R_2$ | $R_3$ und $R_4$ | Umkristallisiert aus Lösungsmittel | Fp. (°C) | Ausbeute (% d. Th.) |
|---|---|---|---|---|---|---|---|
| 66 | $CH_3$ | 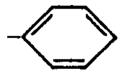 | 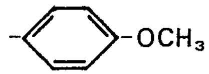 | $(-C_2H_4)_2N-$ 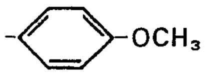 | Methanol/Aceton | 72-76 | 28 |
| 67 | $CH_3$ | 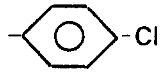 | 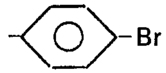 | $(-C_2H_4)_2N-$ 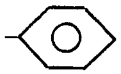 | Äthanol | 149 | 28 |
| 68 | $CH_3$ | 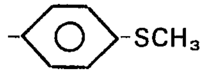 | 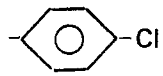 | $-(CH_2)_4-$ | Cyclohexan/Äther | 142-145 | 62 |
| 69 | $CH_2CH_3$ | 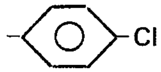 | 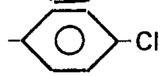 | $-(CH_2)_4-$ | Cyclohexan | 114 | 39 |
| 70 | $CH_2CH_3$ | 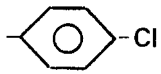 | 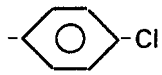 | $(-C_2H_4)_2N-$ 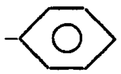 | Äthanol/Äther ×2HCl | 240 | 33 |
| 71 | $CH_3$ | 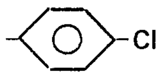 | 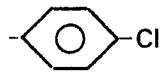 | $(-C_2H_4)_2N-$ 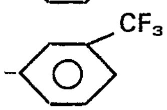 | Äthanol | 214 | 25 |
| 72 | $CH_3$ | 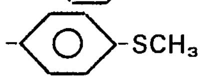 | 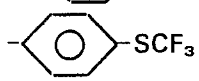 | $(-C_2H_4)_2N-$ 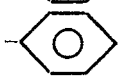 | Äthanol/Äther ×2HCl | 218 | 11 |
| 73 | $CH_3$ | 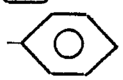 | 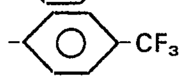 | $(-C_2H_4)_2N-$ 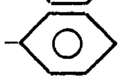 | Cyclohexan | 118-122 | 14 |
| 74 | $CH_3$ | 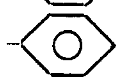 | 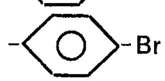 | $(-C_2H_4)_2N-$ 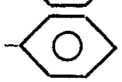 | Petroläther/ Diäthyläther | 110 | 23 |
| 75 | $CH_3$ | 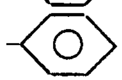 | 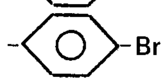 | $-(CH_2)_4-$ | Diäthyläther/ Cyclohexan | 86 | 30 |
| 76 | $CH_3$ | 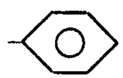 | 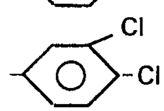 | $(-C_2H_4)_2N-$ 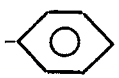 | Äthanol | 145-150 (Zers.) | 31 |
| 77 | $CH_3$ | 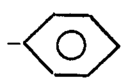 | 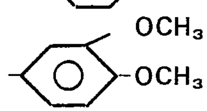 | $(-C_2H_4)_2N-$ 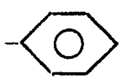 | Äthanol | 125 | 27 |
| 78 | $CH_3$ | 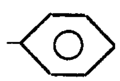 | 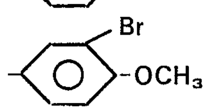 | $(-C_2H_4)_2N-$ 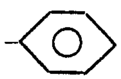 | Petroläther | 65-70 | 40 (amorph) |
| 79 | 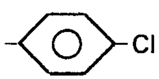 | H | 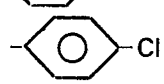 | $(-C_2H_4)_2N-$ 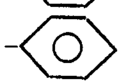 | | | |

**Patentansprüche**

1. Substituierte 2-Amino-3,4-dihydropyridinderivate der allgemeinen Formel (I)

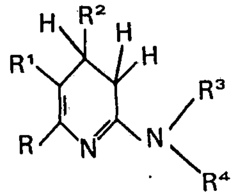

(I)

in welcher

R für geradkettiges oder verzweigtes Alkyl mit bis zu 6 Kohlenstoffatomen steht, welches gegebenenfalls 1- oder 2fach substituiert ist durch Hydroxy, Alkoxy mit 1 bis 4 Kohlenstoffatomen, Amino oder Dialkylamino mit jeweils 1 bis 4 Kohlenstoffatomen, oder für einen Phenyl- oder Benzylrest steht, wobei der Phenylring gegebenenfalls 1- oder 2fach substituiert ist durch gleiche oder verschiedene Substituenten aus der Gruppe Fluor, Chlor, Brom, Trifluormethyl, Alkyl oder Alkoxy mit jeweils 1 bis 4 Kohlenstoffatomen,

$R^1$ und $R^2$ gleich oder verschieden sind und jeweils für Phenyl, Naphthyl, Pyridyl, Furyl oder Thienyl stehen, wobei diese aromatischen Ringe gegebenenfalls durch 1 oder 2 gleiche oder verschiedene Substituenten aus der Gruppe Nitro, Cyano, Hydroxy, Amino, Carboxy, Halogen, Phenyl, Alkyl, Alkoxy, Trifluormethyl, Trifluormethoxy, Trifluormethylthio, Alkylmercapto, Alkylsulfonyl, Alkylamino oder Dialkylamino substituiert sind, wobei die genannten Alkyl- und Alkoxyreste jeweils 1 bis 4 Kohlenstoffatome enthalten, und

$R^3$ und $R^4$ gleich oder verschieden sind und jeweils für Wasserstoff, Benzyl oder Alkyl mit bis zu 6 Kohlenstoffatomen stehen, oder gemeinsam mit dem Stickstoffatom einen 5- bis 7gliedrigen Ring bilden, der gegebenenfalls durch Sauerstoff, NH oder $NR^6$ unterbrochen ist, wobei $R^6$ die für $R^1$ und $R^2$ angegebene Bedeutung besitzt,

sowie ihre pharmazeutisch unbedenklichen Additionssalze.

2. Verbindungen der allgemeinen Formel (I) gemäss Anspruch 1, in welcher

R für Alkyl mit 1 bis 4 Kohlenstoffatomen, Benzyl oder Phenyl steht,

$R^1$ und $R^2$ gleich oder verschieden sind und jeweils für Phenyl, Naphthyl, Pyridyl, Furyl oder Thienyl stehen, wobei diese aromatischen Ringe gegebenenfalls durch 1 oder 2 gleiche oder verschiedene Substituenten aus der Gruppe Cyano, Hydroxy, Carboxy, Halogen, Phenyl, Alkyl, Alkoxy, Trifluormethyl, Trifluormethoxy, Trifluormethylthio, Alkylmercapto, Alkylsulfonyl, Alkylamino oder Dialkylamino substituiert sind, wobei die genannten Alkyl- und Alkoxyreste jeweils 1 bis 4 Kohlenstoffatome enthalten, und

$R^3$ und $R^4$ gleich oder verschieden sind und jeweils für Wasserstoff oder Alkyl mit bis zu 6 Kohlenstoffatomen stehen oder gemeinsam einen 5- bis 7gliedrigen Ring bilden, der gegebenenfalls durch Sauerstoff, NH oder $NR^6$ unterbrochen ist, wobei $R^6$ die für $R^1$ und $R^2$ angegebene Bedeutung besitzt,

sowie ihre pharmazeutisch unbedenklichen Additionssalze.

3. Verfahren zur Herstellung von Verbindungen der allgemeinen Formel (I)

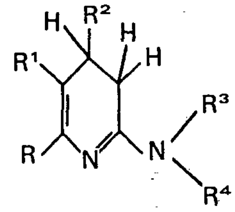

(I)

in welcher

R für geradkettiges oder verzweigtes Alkyl mit bis zu 6 Kohlenstoffatomen steht, welches gegebenenfalls 1- oder 2fach substituiert ist durch Hydroxy, Alkoxy mit 1 bis 4 Kohlenstoffatomen, Amino oder Dialkylamino mit jeweils 1 bis 4 Kohlenstoffatomen, oder für einen Phenyl- oder Benzylrest steht, wobei der Phenylring gegebenenfalls 1- oder 2fach substituiert ist durch gleiche oder verschiedene Substituenten aus der Gruppe Fluor, Chlor, Brom, Trifluormethyl, Alkyl oder Alkoxy mit jeweils 1 bis 4 Kohlenstoffatomen,

$R^1$ und $R^2$ gleich oder verschieden sind und jeweils für Phenyl, Naphthyl, Pyridyl, Furyl oder Thienyl stehen, wobei diese aromatischen Ringe gegebenenfalls durch 1 oder 2 gleiche oder verschiedene Substituenten aus der Gruppe Nitro, Cyano, Hydroxy, Amino, Carboxy, Halogen, Phenyl, Alkyl, Alkoxy, Trifluormethyl, Trifluormethoxy, Trifluormethylthio, Alkylmercapto, Alkylsulfonyl, Alkylamino oder Dialkylamino substituiert sind, wobei die genannten Alkyl- und Alkoxyreste jeweils 1 bis 4 Kohlenstoffatome enthalten, und

$R^3$ und $R^4$ gleich oder verschieden sind und jeweils für Wasserstoff, Benzyl oder Alkyl mit bis zu 6 Kohlenstoffatomen stehen, oder gemeinsam mit dem Stickstoffatom einen 5- bis 7gliedrigen Ring bilden, der gegebenenfalls durch Sauerstoff, NH oder $NR^6$ unterbrochen ist, wobei $R^6$ die für $R^1$ und $R^2$ angegebene Bedeutung besitzt,

sowie ihrer pharmazeutisch unbedenklichen Additionssalze, dadurch gekennzeichnet, dass man α,β-ungesättigte Oxoverbindungen der allgemeinen Formel (II)

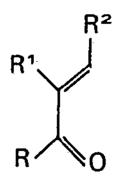

(II)

in welcher

R, $R^1$ und $R^2$ die oben angegebene Bedeutung haben, mit einem 3,3-Diaminoacrylsäureester der allgemeinen Formel (IIIa)

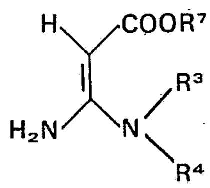

(IIIa)

bzw. ihrer tautomeren Iminform der allgemeinen Formel (IIIb)

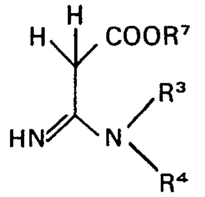

(IIIb)

in welcher

$R^3$ und $R^4$ die oben angegebene Bedeutung haben, und

$R^7$ für einen gegebenenfalls substituierten Alkyl- oder einen Aralkylrest steht, in Gegenwart von inerten organischen Lösungsmitteln und gegebenenfalls von sauren oder basischen Katalysatoren bei Temperaturen zwischen 20 und 150°C umsetzt.

4. Verbindung der allgemeinen Formel (I) gemäss Anspruch 1 zur Verwendung bei der Bekämpfung von Erkrankungen.

5. Verbindung der allgemeinen Formel (I) gemäss Anspruch 1 zur Verwendung bei der Bekämpfung von Erkrankungen des Fettstoffwechsels.

6. Arzneimittel, enthaltend mindestens eine Verbindung der allgemeinen Formel (I) gemäss Anspruch 1.

7. Arzneimittel mit Wirkung auf den Fettstoffwechsel enthaltend mindestens eine Verbindung der allgemeinen Formel (I) gemäss Anspruch 1.

8. Verfahren zur Herstellung von Arzneimitteln gemäss Anspruch 6, dadurch gekennzeichnet, dass man mindestens eine Verbindung der allgemeinen Formel (I) gemäss Anspruch 1, gegebenenfalls unter Verwendung üblicher Hilfs- und Trägerstoffe in eine geeignete Applikationsform überführt.

## Claims

1. Substituted 2-amino-3,4-dihydropyridine derivatives of the general formula (I)

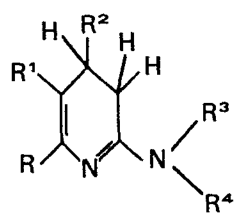

(I)

in which

R represents straight-chain or branched alkyl which has up to 6 carbon atoms and is optionally monosubstituted or disubstituted by hydroxyl, alcoxy with 1 to 4 carbon atoms, amino or dialkylamino with in each case 1 to 4 carbon atoms, or a phenyl or benzyl radical, the phenyl ring optionally being monosubstituted or disubstituted by identical or different substituents from the group comprising fluorine, chlorine, bromine, trifluoromethyl, and alkyl and alcoxy with in each case 1 to 4 carbon atoms,

$R^1$ and $R^2$ are identical or different and each represents phenyl, naphthyl, pyridyl, furyl or thienyl, these aromatic rings optionally being substituted by one or two identical or different substituents from the group comprising nitro, cyano, hydroxyl, amino, carboxyl, halogen, phenyl, alkyl, alcoxy, trifluoromethyl, trifluoromethoxy, trifluoromethylthio, alkylmercapto, alkylsulphonyl, alkylamino and dialkylamino and the alkyl and alcoxy radicals mentioned each containing 1 to 4 carbon atoms, and

$R^3$ and $R^4$ are identical or different and each represents hydrogen, benzyl or alkyl with up to 6 carbon atoms, or $R^3$ and $R^4$ together with the nitrogen atom, form a 5-membered to 7-membered ring which is optionally interrupted by oxygen, NH or $NR^6$, wherein $R^6$ has the meaning indicated for $R^1$ and $R^2$,

and their pharmaceutically acceptable addition salts.

2. Compounds of the general formula (I) according to claim 1, in which

R represents alkyl with 1 to 4 carbon atoms, benzyl or phenyl,

$R^1$ and $R^2$ are identical or different and each represents phenyl, naphthyl, pyridyl, furyl or thienyl, these aromatic rings optionally being substituted by one or two identical or different substituents from the group comprising cyano, hydroxyl, carboxyl, halogen, phenyl, alkyl, alcoxy, trifluoromethyl, trifluoromethoxy, trifluoromethylthio, alkylmercapto, alkylsulphonyl, alkylamino and dialkylamino and the alkyl and alcoxy radicals mentioned each containing 1 to 4 carbon atoms, and

$R^3$ and $R^4$ are identical or different and each represents hydrogen or alkyl with up to 6 carbon atoms, or $R^3$ and $R^4$ together form a 5-membered to 7-membered ring which is optionally interrupted by oxygen, NH or $NR^6$, wherein $R^6$ has the meaning indicated for $R^1$ and $R^2$,

and their pharmaceutically acceptable addition salts.

3. Process for the preparation of compounds of the general formula (I)

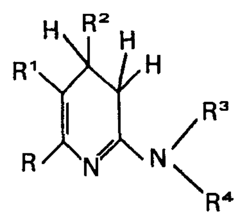

(I)

in which

R represents straight-chain or branched alkyl which has up to 6 carbon atoms and is optionally monosubstituted or disubstituted by hydroxyl, alcoxy with 1 to 4 carbon atoms, amino or dialkylamino with in each case 1 to 4 carbon atoms, or a phenyl or benzyl radical, the phenyl ring optionally being monosubstituted or disubstituted by identical or different substituents from the group comprising fluorine, chlorine, bromine, trifluoromethyl, and alkyl and alcoxy with in each case 1 to 4 carbon atoms,

$R^1$ and $R^2$ are identical or different and each represents phenyl, naphthyl, pyridyl, furyl or thienyl, these aromatic rings optionally being

substituted by one or two identical or different substituents from the group comprising nitro, cyano, hydroxyl, amino, carboxyl, halogen, phenyl, alkyl, alcoxy, trifluoromethyl, trifluoromethoxy, trifluoromethylthio, alkylmercapto, alkylsulphonyl, alkylamino and dialkylamino and the alkyl and alcoxy radicals mentioned each containing 1 to 4 carbon atoms, and

$R^3$ and $R^4$ are identical or different and each represents hydrogen, benzyl or alkyl with up to 6 carbon atoms, or $R^3$ and $R^4$, together with the nitrogen atom, form a 5-membered to 7-membered ring which is optionally interrupted by oxygen, NH or $NR^6$, wherein $R^6$ has the meaning indicated for $R^1$ and $R^2$,

and their pharmaceutically acceptable addition salts, characterized in that α,β-unsaturated oxo compounds of the general formula (II)

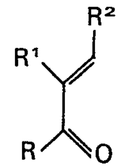

(II)

in which

R, $R^1$ and $R^2$ have the meaning indicated above, are reacted with a 3,3-diaminoacrylic acid ester of the general formula (IIIa)

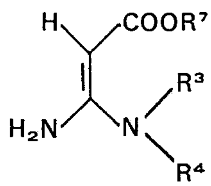

(IIIa)

or with its tautomeric imine form of the general formula (IIIb)

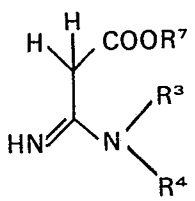

(IIIb)

in which

$R^3$ and $R^4$ have the meaning indicated above, and

$R^7$ represents an optionally substituted alkyl or aralkyl radical, in the presence of inert organic solvents and if appropriate of acid or basic catalysts, at temperatures between 20 and 150°C.

4. Compounds of the general formula (I) according to claim 1 for use in combating diseases.

5. Compounds of the general formula (I) according to claim 1 for use in combating disorders in fat metabolism.

6. Medicaments containing at least one compound of the general formula (I) according to claim 1.

7. Medicaments with an action on fat metabolism containing at least one compound of the general formula (I) according to claim 1.

8. Process for the preparation of medicaments according to claim 6, characterized in that at least one compound of the general formula (I) according to claim 1 is converted into a suitable form of administration, if appropriate using customary auxiliaries and excipients.

## Revendications

1. Dérivés substitués de 2-amino-3,4-dihydropyridine de formule générale (I)

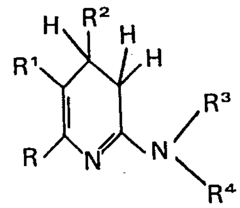

(I)

dans laquelle

R est un groupe alkyle à chaîne droite ou ramifiée ayant jusqu'à 6 atomes de carbone, qui est éventuellement substitué une ou deux fois par un radical hydroxy, alcoxy ayant 1 à 4 atomes de carbone, amino ou dialkylamino ayant 1 à 4 atomes de carbone par radical alkyle, ou bien un ester phényle ou benzyle, le noyau phényle étant éventuellement substitué une ou deux fois par des substituants égaux ou différents choisis dans le groupe comprenant le fluor, le chlore, le brome, le radical trifluorométhyle, un radical alkyle ou alcoxy ayant chacun 1 à 4 atomes de carbone,

$R^1$ et $R^2$ sont égaux ou différents et représentent chacun un radical phényle, naphtyle, pyridyle, furyle ou thiényle, ces noyaux aromatiques étant éventuellement substitués par un ou deux substituants égaux ou différents choisis dans le groupe comprenant les radicaux nitro, cyano, hydroxy, amino, carboxy, halogéno, phényle, alkyle, alcoxy, trifluorométhyle, trifluorométhoxy, trifluorométhylthio, alkylmercapto, alkylsulfonyle, alkylamino ou dialkylamino, les restes alkyle et alcoxy mentionnés comportant chacun 1 à 4 atomes de carbone, et

$R^3$ et $R^4$ sont égaux ou différents et représentent chacun l'hydrogène, le radical benzyle ou un radical alkyle ayant jusqu'à 6 atomes de carbone, ou forment conjointement avec l'atome d'azote un noyau pentagonal à heptagonal qui est éventuellement interrompu par de l'oxygène, le groupe NH ou un groupe $NR^6$, $R^6$ ayant la définition indiquée pour $R^1$ et $R^2$,

ainsi que leurs sels d'addition pharmaceutiquement acceptables.

2. Composés de formule générale (I) suivant la revendication 1, dans laquelle

R est un radical alkyle ayant 1 à 4 atomes de carbone, benzyle ou phényle,

$R^1$ et $R^2$ sont égaux ou différents et représentent chacun un radical phényle, naphtyle, pyridyle, furyle ou thiényle, ces noyaux aromatiques étant éventuellement substitués par un ou deux substituants égaux ou différents choisis dans le groupe des radicaux cyano, hydroxy, carboxy, halogéno, phényle, alkyle, alcoxy, trifluorométhyle, trifluorométhoxy, trifluorométhylthio, alkylmercapto, alkylsulfonyle, alkylamino ou dialkylamino, les

restes alkyle et alcoxy mentionnés contenant chacun 1 à 4 atomes de carbone, et

$R^3$ et $R^4$ sont égaux ou différents et représentent chacun l'hydrogène ou un radical alkyle ayant jusqu'à 6 atomes de carbone, ou forment conjointement un noyau pentagonal à heptagonal qui est éventuellement interrompu par de l'oxygène, le groupe NH ou un groupe $NR^6$, $R^6$ possédant la définition indiquée pour $R^1$ et $R^2$,

ainsi que leurs sels d'addition pharmaceutiquement acceptables.

3. Procédé de préparation de composés de formule générale (I)

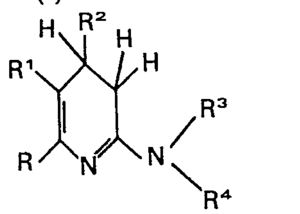

(I)

dans laquelle

R est un groupe alkyle à chaîne droite ou ramifiée ayant jusqu'à 6 atomes de carbone, qui est éventuellement substitué une ou deux fois par un radical hydroxy, alcoxy ayant 1 à 4 atomes de carbone, amino ou dialkylamino ayant 1 à 4 atomes de carbone par radical alkyle, ou bien un reste phényle ou benzyle, le noyau phényle étant éventuellement substitué une ou deux fois par des substituants égaux ou différents choisis dans le groupe comprenant le fluor, le chlore, le brome, le radical trifluorométhyle, un radical alkyle ou alcoxy ayant chacun 1 à 4 atomes de carbone,

$R^1$ et $R^2$ sont égaux ou différents et représentent chacun un radical phényle, naphtyle, pyridyle, furyle ou thiényle, ces noyaux aromatiques étant éventuellement substitués par un ou deux substituants égaux ou différents choisis dans le groupe comprenant les radicaux nitro, cyano, hydroxy, amino, carboxy, halogéno, phényle, alkyle, alcoxy, trifluorométhyle, trifluorométhoxy, trifluorométhylthio, alkylmercapto, alkylsulfonyle, alkylamino ou dialkylamino, les restes alkyle et alcoxy mentionnés comportant chacun 1 à 4 atomes de carbone, et

$R^3$ et $R^4$ sont égaux ou différents et représentent chacun l'hydrogène, le radical benzyle ou un radical alkyle ayant jusqu'à 6 atomes de carbone, ou forment conjointement avec l'atome d'azote un noyau pentagonal à heptagonal qui est éventuellement interrompu par de l'oxygène, le groupe NH ou un groupe $NR^6$, $R^6$ ayant la définition indiquée pour $R^1$ et $R^2$,

ainsi que de leurs sels d'addition pharmaceutiquement acceptables, caractérisé en ce qu'on fait réagir à des températures comprises entre 20 et 150° C, en présence de solvants organiques inertes et, le cas échéant, de catalyseurs acides ou basiques, des composés oxo à non-saturation α,β de formule générale (II)

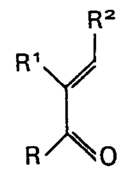

(II)

dans laquelle R, $R^1$ et $R^2$ ont la définition indiquée ci-dessus, avec un ester d'acide 3,3-diaminoacrylique de formule générale (IIIa)

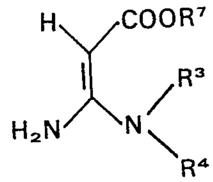

(IIIa)

ou sa forme imine tautomère de formule générale (IIIb)

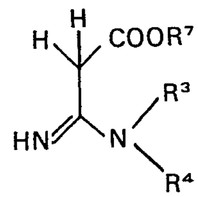

(IIIb)

dans laquelle

$R^3$ et $R^4$ ont la définition indiquée ci-dessus, et

$R^7$ est un reste alkyle éventuellement substitué ou un reste aralkyle.

4. Composés de formule générale (I) suivant la revendication 1, destinés à être utilisés pour combattre des maladies.

5. Composés de formule générale (I) suivant la revendication 1, destinés à être utilisés pour combattre des maladies du métabolisme.

6. Médicament contenant au moins un composé de formule générale (I) suivant la revendication 1.

7. Médicament agissant sur le métabolisme, contenant au moins un composé de formule générale (I) suivant la revendication 1.

8. Procédé de préparation de médicaments suivant la revendication 6, caractérisé en ce qu'on transforme au moins un composé de formule générale (I) suivant la revendication 1 en une forme d'administration appropriée, en utilisant éventuellement des adjuvants et des supports classiques.